(19) Europäisches Patentamt
European Patent Office
Office européen des brevets

(11) Numéro de publication: **0 223 765 A1**

(12) **DEMANDE DE BREVET EUROPEEN**

(21) Numéro de dépôt: **86870145.9**

(22) Date de dépôt: **09.10.86**

(51) Int. Cl.⁴: **C 07 C 49/17**, C 07 C 49/24, C 12 P 7/02, C 12 P 7/04, C 12 P 7/26, C 12 R 1/865

(30) Priorité: **11.10.85 FR 8515103**

(43) Date de publication de la demande: **27.05.87 Bulletin 87/22**

(84) Etats contractants désignés: **AT BE CH DE ES FR GB GR IT LI LU NL SE**

(71) Demandeur: **SANOFI, société anonyme, 40, Avenue George V, F-75008 Paris (FR)**
Demandeur: **CENTRE NATIONAL DE LA RECHERCHE SCIENTIFIQUE (CNRS), 15, Quai Anatole France, F-75700 Paris (FR)**

(72) Inventeur: **Veschambre, Henri, 33bis rue Cotepet, F-63000 Clermont-Ferrand (FR)**
Inventeur: **Bolte, Jean, F-63117 Chauriat (FR)**
Inventeur: **Gourcy, Jean Gabriel, 78bis avenue de la Gare, F-63360 Gerzat (FR)**
Inventeur: **Pigerol, Charles, 13, Parc du Béarn, F-92210 Saint-Cloud (FR)**

(74) Mandataire: **Cauchie, Daniel, c/o S.A. LABAZ-SANOFI N.V. Avenue De Béjar, 1, B-1120 Bruxelles (BE)**

(54) **Procédé de préparation de dérivés hydroxy-3 cetoniques.**

(57) L'invention se rapporte à un procédé de préparation de composés comportant la séquence acyclique de formule:

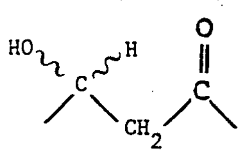

procédé suivant lequel on effectue, à une température de 30 à 37°C, la réduction de la séquence acyclique dicétone-1,3 énolisable de formule:

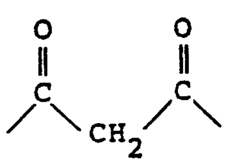

au moyen de *Saccharomyces cerevisiae*.

EP 0 223 765 A1

PROCEDE DE PREPARATION DE DERIVES HYDROXY-3 CETONIQUES

La présente invention se rapporte, d'une manière générale, à la préparation de dérivés cétols et, en particulier, à la préparation de composés comportant la séquence acyclique hydroxy-3 cétonique de formule :

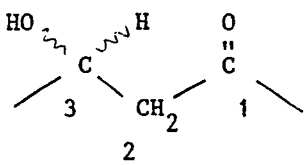

En conséquence, l'invention se rapporte notamment à la préparation de composés hydroxy-3 cétone de formule générale :

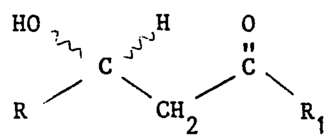

I

dans laquelle R et $R_1$, identiques ou différents représentent un radical organique par exemple un radical hydrocarboné, linéaire ou ramifié, aromatique ou alicyclique.

Comme exemples de valeurs pour R et $R_1$ on peut citer un radical alkyle, alkényle ou alkynyle ayant de 1 à 10 atomes de carbone tels qu'un radical méthyle, éthyle, n-propyle, isopropyle, n-butyle, isobutyle, tertiobutyle, n-pentyle, n-hexyle, n-heptyle, n-decyle, allyle ou pentèn-4 yle.

La réduction microbiologique des cétones est un procédé déjà ancien qui a permis d'obtenir de nombreux alcools chiraux avec une grande pureté optique. La réduction d'α-chloro et d'α-hydroxycétones a également été étudiée et a conduit à des composés bifonctionnels chiraux. Enfin, la réduction de β-céto-esters a fait l'objet de nombreux travaux, tant pour son application à la synthèse de produits naturels que pour l'étude de la stéréochimie de la réaction .

Dans la série des dicétones-1,3 , seule la réduction microbiologique de dicétones-1,3 cycliques non énolisables et comportant un substituant en position 2 a été observée [Tetrahedron Lett.,25, 1241(1984)] en utilisant comme microorganisme la levure de boulanger (<u>Saccharomyces cerevisiae</u>).

Au cours de l'élaboration de l'invention, on a appliqué cette méthode pour la réduction de dicétones acycliques peu énolisées et comportant un substituant en position 2 notamment pour la réduction de la méthyl-3 pentanedione-2,4.

Cette réduction s'est avérée très difficile : après 6 jours de réaction on a retrouvé 70% de produit de départ.

Or, on a trouvé, dans le cadre de l'invention, que cette réduction microbiologique s'accomplit avec grande facilité et rendements excellents lorsque la dicétone de départ est une dicétone-1,3 acyclique énolisable, c'est-à-dire ne comportant aucun substituant en position 2.

Ainsi, selon l'invention, on prépare des composés comportant la séquence acyclique hydroxy-3 cétonique de formule :

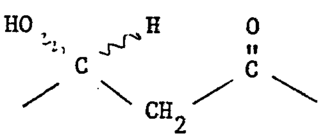

en effectuant la réduction de composés comportant la séquence acyclique dicétone-1,3 énolisable de formule :

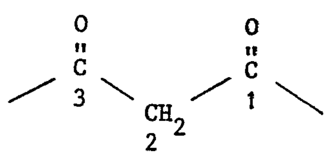

au moyen de Saccharomyces cerevisiae et à une température de 30 à 37°C de préférence à 35°C.

Ce procédé convient particulièrement pour la préparation des composés hydroxy-3 cétone de formule I ci-dessus à partir de composés dicétones-1,3 de formule générale :

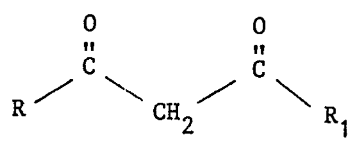

II

dans laquelle R et $R_1$ ont la signification donnée précédemment.

Sur le plan pratique, d'une manière générale, on effectue la réduction en introduisant la dicétone-1,3 de départ dans une suspension maintenue à 35°C constituée de levure de boulanger (Saccharomyces cerevisiae) en milieu

nutritif par exemple une solution aqueuse de saccharose. On agite le mélange toute la durée de la réaction et toutes les 24 heures on réintroduit une nouvelle quantité de saccharose. De cette manière on obtient le dérivé hydroxy-3 cétonique désiré en 3 jours en moyenne.

La pureté optique du cétol produit par le procédé de l'invention a été déterminée par chromatographie en phase gazeuse après réduction en diol par le borohydrure de sodium. On traite alors le mélange de diols ainsi obtenu au moyen d'une solution d'isocyanate de méthyle dans le chlorure de méthylène. Après évaporation de l'excès de réactif, on injecte le mélange non purifié d'uréthanes sur une colonne capillaire chirale de 25 m. On réalise également un essai témoin avec le mélange de diols obtenu par réduction de la dicétone-1,3 correspondante, au moyen de borohydrure de sodium.

A titre d'exemples, on a consigné ci-dessous les résultats de pureté optique du cétol obtenu après réduction de l'acétylacétone, l'hexanedione-2,4, le dioxo-2,4 nonène-8 et l'heptanedione-3,5.

1) <u>(+)(2S)-hydroxy-2 pentanone-4</u>

L'acétylacétone est transformée en (+)(2S)-hydroxy-2 pentanone-4, produit connu selon Bull. Soc. Chem. Jap., 46, 2233 (1973). Ce composé est obtenu selon l'invention avec une très grande pureté optique. L'analyse en chromatographie phase vapeur du pentanediol obtenu par réduction chimique de l'acétylacétone fait apparaître 3 pics dans les proportions 25/25/50. Les deux premiers sont attribués aux énantiomères, le troisième au dérivé <u>méso</u>. Si le cétol obtenu par le procédé de l'invention est optiquement pur, la réduction par le borohydrure de sodium doit conduire à un mélange de 2 diols : un énantiomère (2S,4S) et le dérivé <u>méso</u> (2S,4R).

Ces deux isomères sont effectivement observés lors de l'analyse, l'isomère (2S,4S) étant celui des deux énantiomères qui a le plus long temps de rétention.

2) <u>(+)(2S)-hydroxy-2 hexanone-4 et (+)(2S)-hydroxy-2 oxo-4 nonène-8</u>

La réaction est plus rapide avec l'hexanedione-2,4 et le dioxo-2,4 nonène-8 qu'avec l'acétylacétone, l'augmentation du caractère lipophile du substrat favorisant la vitesse de réduction. On a obtenu dans chaque cas le cétol ayant la fonction alcool en position 2, c'est-à-dire l'hydroxy-2 hexanone-4 et l'hydroxy-2 oxo-4 nonène-8.

Aucune trace d'hydroxy en position 4 n'a été observée comme le montrent

les spectres de résonance magnétique nucléaire (R.M.N.) repris dans l'Exemple ci-après. La configuration S a été attribuée à ces deux cétols étant donné que le pouvoir rotatoire de l'hydroxy-2 hexanone-4 et de l'hydroxy-2 oxo-4 nonène-8 est du même signe que celui de la (+)(2S)-hydroxy-2 pentanone-4.

L'analyse en chromatographie phase vapeur des diols obtenus par réduction chimique de l'hydroxy-2 hexanone-4 et de l'hydroxy-2 oxo-4 nonène-8 ne montre qu'un seul pic pour chacun de ces produits dans la région des énantiomères RR et SS.

Comme dans le cas de la (+)(2S)-hydroxy-2 pentanone-4, ce pic correspond à celui des deux qui a le temps de rétention le plus long, c'est-à-dire SS. Il n'y a aucune trace de RR ce qui prouve la très grande pureté optique de la (+)(2S)-hydroxy-2 hexanone-4 et du (+)(2S)-hydroxy-2 oxo-4 nonène-8.

3) (-)(3R)-hydroxy-3 heptanone-5

On a attribué la configuration R au carbone asymétrique étant donné l'inversion du signe du pouvoir rotatoire de l'hydroxy-3 heptanone-5 obtenue par rapport à celui des cétols précédents. L'application de la méthode de HOREAU [Tetrahedron Lett., 21, 965 (1962)] indique que l'alcool a la configuration R. La réduction par le borhydrure de sodium de l'hydroxy-3 heptanone-5 obtenue par le procédé de l'invention fournit trois diols, dont un méso, le mélange ainsi obtenu présentant un pouvoir rotatoire négatif. Or l'heptanediol-3,5 optiquement actif est connu selon Bull. Soc. Chem. Jap. 53, 3367 (1980) et la configuration (3R,5R) a été attribuée dans cette publication au diol de pouvoir rotatoire négatif. L'analyse chromatographique de ce diol sur colonne chirale permet de déterminer la pureté optique de la (-)(3R)-hydroxy-3 heptanone-5. On obtient deux pics dans la région des énantiomères RR et SS, le pic le plus abondant étant celui qui présente le plus faible temps de rétention contrairement aux cétols précédents. On observe, cependant, une plus faible spécificité de cette réduction que dans les cas évoqués précédemment.

Ces résultats montrent que la réduction de dicétones-1,3 acycliques permet de préparer très facilement des cétols avec généralement une très grande pureté optique. Ces cétols sont des synthons intéressants. En particulier, ils peuvent être réduits en diols optiquement purs en utilisant la méthode de NARASAKA et coll. [Tetrahedron, 40, 2238 (1984)].

Ces diols sont des produits de départ de synthèse de composés naturels [Helv. Chim. Acta, 57, 2306 (1974)] et en particulier de phéromones [Tetrahedron Lett., 25, 2175 (1984)].

En conséquence, les composés préparés par le procédé de l'invention constitueront des produits intermédiaires intéressants notamment la (+)(2S)-hydroxy-2 hexanone-4, le (+)(2S)-hydroxy-2 oxo-4 nonène-8 et la (-)(3R)-hydroxy-3 heptanone-5 qui sont des produits nouveaux.

Ces composés nouveaux représentent un autre objet de l'invention.

L'Exemple non limitatif suivant illustre l'invention :

EXEMPLE

Préparation de dérivés hydroxy-3 cétone-1

On met en suspension 200g de levure de boulanger (*Saccharomyces cerevisiae*) dans 4 l d'une solution aqueuse de saccharose (30g par litre).

On maintient le mélange à 35°C et on agite pendant toute la durée de la réaction. Après 30 min., on ajoute 4g de dicétone-1,3. Toutes les 24 heures, on ajoute 50g de saccharose. Lorsque la réaction est terminée, on filtre, on sature le filtrat avec du sulfate d'ammonium et on extrait à l'éther éthylique. Après séchage et évaporation du solvant, on obtient un résidu que l'on purifie par distillation ou par chromatographie sur colonne de gel de silice, ce qui fournit le dérivé hydroxy-3 cétone-1 désiré.

En utilisant ce procédé, on a préparé les composés suivants :

a) à partir d'acétylacétone et après 6 jours de réaction, la (+)(2S)-hydroxy-2 pentanone-4

Rendement : 90% (avant purification)

Excès énantiomérique : > 99%

$\alpha_D^{25}$ : + 55° (C = 0,05, chloroforme)

b) à partir d'hexanedione-2,4 et après 3 jours de réaction, la (+)(2S)-hydroxy-2 hexanone-4.

Rendement : 100% (avant purification)

Excès énantiomérique : > 99%

$\alpha_D^{25}$ : + 63° (C = 0,09, chloroforme)

Spectre R.M.N. $^1H$ (60 MHz, $CDCl_3$) $\delta$ ppm : 4,1 (s, 1H échangeable avec $D_2O$) ; 4,6 à 4 (m, 1H) ; 2,6 (d, 2H, J = 6Hz); 2,6 à 2,2 (m, 2H); 1,20 (d, 3H, J = 6Hz); 1,05 (t, 3H, J = 7Hz).

c) à partir de dioxo-2,4 nonène-8 et après 3 jours de réaction, le (+)(2S)-

hydroxy-2 oxo-4 nonène-8
Rendement : 100% (avant purification)
Excès énantiomérique : > 99%
$\alpha_D^{25}$ : + 55,5° (C = 0,06, chloroforme)
Spectre R.M.N. $^1H$ (60 MHz, $CDCl_3$) $\delta$ ppm : 6,1 à 5,5 (m, 1H); 5,3 à 4,8 (m, 2H); 4,5 à 4,1 (m, 1H); 3,15 (s, 1H échangeable avec $D_2O$); 2,60 à 1,40 (m, 8H); 1,20 (d, 3H, J = 7Hz)

d) à partir d'heptanedione-3,5 et après 3 jours de réaction, la (-)(3R)-hydroxy-3 heptanone-5
Rendement : 100% (avant purification)
$\alpha_D^{25}$ : -13,5° (C = 0,08, chloroforme)
Spectre R.M.N. $^1H$ (200 MHz, $CDCl_3$) $\delta$ ppm : 4,05 à 4,20 et 3,80 à 4 (m, 0,8H et 0,2H); 3,14 (s, échangeable avec $D_2O$); 2,45 à 2,60 (m, 1H); 2,21 (s, 3H); 1,08 à 1,24 (m, 6H).

REVENDICATIONS

1. Procédé de préparation de composés comportant la séquence acyclique de formule :

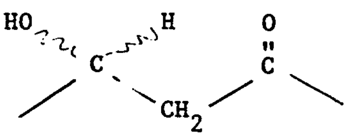

caractérisé en ce que l'on effectue, à une température de 30 à 37°C, la réduction de la séquence acyclique dicétone-1,3 énolisable de formule :

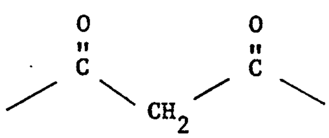

au moyen de Saccharomyces cerevisiae.

2. Procédé de préparation de composés hydroxy-3 cétones de formule générale :

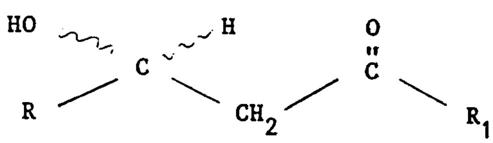

dans laquelle R et $R_1$, identiques ou différents, représentent un radical hydrocarboné linéaire ou ramifié, aromatique ou alicyclique caractérisé en ce que l'on effectue, à une température de 30 à 37°C la réduction de composés dicétones-1,3 de formule générale :

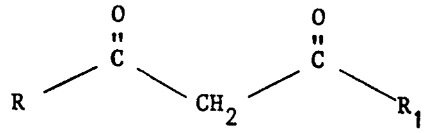

dans laquelle R et $R_1$ ont la même signification que précédemment, au moyen de Saccharomyces cerevisiae.

3. Procédé de préparation de composés hydroxy-3 cétone selon la Revendication 2 caractérisé en ce que R et $R_1$, identiques ou différents, représentent un radical alkyle, alkényle ou alkynyle ayant de 1 à 10 atomes de carbone.
4. (+)(2S)-hydroxy-2 hexanone-4
5. (+)(2S)-hydroxy-2 oxo-4 nonène-8
6. (-)(3R)-hydroxy-3 heptanone-5.

Office européen des brevets

**RAPPORT DE RECHERCHE EUROPEENNE**

0223765

Numéro de la demande

EP 86 87 0145

**DOCUMENTS CONSIDERES COMME PERTINENTS**

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 92, 1980, page 545, résumé no. 93901g, Columbus, Ohio, US; & JP-A-79 103 811 (TEIJIN LTD) 15-08-1979<br>* Résumé *<br>--- | 4 | C 07 C 49/17<br>C 07 C 49/24<br>C 12 P 7/02<br>C 12 P 7/04<br>C 12 P 7/26<br>C 12 R 1/865 |
| X | CHEMICAL ABSTRACTS, vol. 89, 1978, page 548, résumé no. 42503j, Columbus, Ohio, US; R. LE GOALLER et al.: "Cyclopropanediols. VII. Chemical behavior of cyclopropanediols and reactions of 1,2-bis(trimethylsiloxy)cyclopropanes", & CAN. J. CHEM. 1978, 56(4), 481-6<br>* Résumé *<br>--- | 6 | DOMAINES TECHNIQUES RECHERCHES (Int. Cl.4) |
| D,Y | TETRAHEDRON LETTERS, vol. 25, no. 12, 1984, pages 1241-1244, Pergamon Press Ltd, GB; D.W. BROOKS et al.: "Chiral cyclohexanoid synthetic precursors via asymmetric microbial reduction of prochiral cyclohexaned iones"<br>* Pages 1241-1242 *<br>--- | 1 | C 07 C 49/00<br>C 12 P 7/00<br>C 12 R 1/00 |
| Y | FR-A-2 060 121 (SYNTEX CORP.)<br>* Revendications *<br>--- -/- | 1 | |

Le présent rapport de recherche a été établi pour toutes les revendications

| Lieu de la recherche | Date d'achèvement de la recherche | Examinateur |
|---|---|---|
| LA HAYE | 19-01-1987 | BONNEVALLE E.I.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

# RAPPORT DE RECHERCHE EUROPEENNE

0223765

Numéro de la demande

EP 86 87 0145



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| P,Y | US-A-4 584 193 (W.E. BURKHOLDER et al.)<br>* Colonne 2 *<br>----- | 3 | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 19-01-1987 | BONNEVALLE E.I.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82